# EUROPÄISCHE PATENTSCHRIFT

(11) **EP 2 611 430 B1**
(45) Veröffentlichungstag und Bekanntmachung des Hinweises auf die Patenterteilung: **12.03.2014**
(21) Anmeldenummer: 12743174.0
(22) Anmeldetag: 03.08.2012
(51) Int. Cl.: A61K 9/70, B65H 35/00, B65H 37/00, A61F 13/02

(54) **SCHUTZFOLIENWECHSLER**
PROTECTIVE-FILM CHANGER
CHANGEUR DE FEUILLE DE PROTECTION

(30) Priorität: 03.08.2011 DE 102011080389
(43) Veröffentlichungstag der Anmeldung: 10.07.2013
(73) Patentinhaber: Acino AG, 83714 Miesbach (DE)
(72) Erfinder: GRADER, Ludwig, 56626 Andernach (DE); SCHURAD, Björn, 81667 München (DE); PIOTROWSKI, Holger, 83727 Schliersee (DE)
(74) Vertreter: Beetz & Partner
(86) Internationale Anmeldenummer: PCT/EP2012/065249
(87) Internationale Veröffentlichungsnummer: WO 2013/017686

(56) Entgegenhaltungen:
- EP-A1- 1 967 171
- WO-A1-00/03919
- WO-A1-01/28904
- US-B1- 7 029 549

## Beschreibung

Die vorliegende Erfindung bezieht sich auf die Herstellung transdermaler therapeutischer Systeme und insbesondere auf ein Anbringen von Schutzabdeckungen an transdermale therapeutische Systeme.

Transdermale therapeutische Systeme werden zum Verabreichen eines in ihnen enthaltenen Wirkstoffs in der Regel auf der Haut eines Patienten angebracht. Die Verabreichung erfolgt durch Diffusion des Wirkstoffs in die Haut und über die darin vorhandenen Gefäße in den Blutkreislauf des Patienten.

Transdermale therapeutische Systeme umfassen gewöhnlich ein Wirkstoffdepot, eine wirkstoffundurchlässige Rückschicht zum Abdecken der nicht zum Kontakt mit der Haut vorgesehenen Seite des Wirkstoffdepots und eine Schutzfolie zum Abdecken der zum Kontakt mit der Haut vorgesehenen Applikationsseite des Wirkstoffdepots. Die Schutzfolie ist an einer selbstklebend ausgeführten Oberfläche des Wirkstoffdepots befestigt. Unter selbstklebend ist hierbei das Vermögen zu verstehen, bei Kontakt mit einer Oberfläche eines Gegenstands an dieser anzuhaften, wobei eine solche adhäsive Verbindung ohne substantielle Beeinträchtigung der beiden Adhäsionspartner wieder gelöst werden kann. Der Begriff selbstklebend wird in dieser Schrift somit synonym zum Begriff haftklebend verwendet. Die Schutzfolie dient dem Schutz der Applikationsseite vor Verschmutzung und anderen Außeneinwirkungen sowie dem Schutz der Umgebung des transdermalen therapeutischen Systems vor Kontamination durch den Wirkstoff. Die Schutzfolie wird vor der Applikation des Systems entfernt.

Bei der Herstellung transdermaler therapeutischer Systeme mit ganzflächig oder weitgehend ganzflächig selbstklebenden Applikationsseiten wird das Wirkstoffdepot zunächst bahnförmig auf eine Trägerfolie aufgebracht. Das Wirkstoffdepot kann dabei schmäler als die Trägerfolie sein und auch in mehreren zueinander beabstandeten Bahnen auf die Trägerfolie aufgebracht werden. Unter Bahn ist in dieser Schrift ein Gebilde mit begrenzter Breite und nicht näher spezifizierter Länge zu verstehen. An der von der Trägerfolie abgewandten Oberseite kann das Wirkstoffdepot eventuell mit einer wirkstoffundurchlässigen Rückschicht bedeckt sein. Das bahnförmig auf die Trägerfolie aufgebrachte Wirkstoffdepot bzw. Laminat von Wirkstoffdepot und Rückschicht wird im Folgenden auch als Pflasterbahn bezeichnet.

Abhängig vom jeweils mit einem transdermalen therapeutischen System zu verabreichenden Wirkstoff bzw. zu verabreichender Wirkstoffkombination werden unterschiedlich ausgebildete Wirkstoffdepots bevorzugt. Gegenwärtig sind zwei Grundtypen transdermaler therapeutischer Systeme bekannt, Matrixsysteme und Reservoirsysteme. Bei den sogenannten Matrixsystemen ist der Wirkstoff in einer Polymermatrix enthalten, die zumeist aus einem selbstklebenden bzw. -haftenden, druckempfindlichen Polymer gebildet ist. Die Wirkstoffabgabe wird über das Konzentrationsgefälle zur Haut gesteuert. Bei den sogenannten Reservoirsystemen ist der Wirkstoff in einem flüssigen, halbfesten oder festen Reservoir enthalten, wobei die Wirkstoffabgabe üblicherweise mittels einer Membran reguliert wird.

Zum Erhalt einzelner transdermaler therapeutischer Systeme, werden in die Pflasterbahn Trennlinien eingebracht, die sich von der Oberfläche der Pflasterbahn bis zur Trägerfolie erstrecken und die Konturen der einzelnen Pflaster definieren. In der Regel sind die einzelnen Trennlinien in sich geschlossen und trennen die einzelnen Pflaster so vom übrigen Teil der Pflasterbahn ab. Häufig weist der nicht für Pflaster nicht genutzte Teil der Pflasterbahn eine zusammenhängende Struktur auf, um leichter von der Trägerfolie abgezogen werden zu können.

Bei einigen Systemen bildet die Trägerfolie gleichzeitig die Schutzfolie. Um ein Ablösen der Schutzfolie bei der Applikation eines transdermalen therapeutischen Systems zu erleichtern, wird diese häufig zweiteilig ausgeführt, wozu die Trägerfolie in Bahnrichtung geschlitzt wird. Der Schlitz führt unter den einzelnen Pflastern hindurch, so dass jedes der Pflaster auf beiden Schutzfolienteilen aufliegt. Eine derartiges Vorgehen eignet sich nicht für die Herstellung transdermaler therapeutischer Systeme mit sich überlappenden bzw. unterschiedlich ausgebildeten Schutzfolienteilen. Solche Systeme erfordern ein Überführen der Pflaster auf Schutzfolienteile, die von der ursprünglichen Trägerfolie verschieden sind.

Die Patentschrift DE 44 06 976 C1 beschreibt ein Spenderverfahren, bei dem ursprünglich auf einer ersten Bahn befindliche selbstklebende Abschnitte von dieser auf eine zweite Bahn überführt werden. Ein solches Verfahren ermöglicht ein Überführen von Pflastern von einer Trägerfolie auf eine von dieser verschiedene Schutzfolie. Zum Übertragen der selbstklebenden Abschnitte wird die erste Bahn über eine senkrecht zur Bahnrichtung angeordnete Spenderkante geführt und dort spitzwinkelig umgelenkt. Beim Umlenken löst sich die erste Bahn von den selbstklebenden Abschnitten aufgrund deren Eigensteifigkeit ab und letztere gelangen dann auf die nahe der Spenderkante herangeführte zweite Bahn, worauf sie schließlich haften bleiben.

Moderne transdermale therapeutische Systeme weisen jedoch häufig nicht die Eigensteifigkeit auf, die zum Ablösen der Trägerfolienbahn an der Spenderkante erforderlich ist, da sie im Hinblick auf einen hohen Tragekomfort nicht nur sehr flexibel, sondern auch elastisch ausgeführt sind.

Bei den in der Offenlegungsschrift DE 199 46 384 A1 beschriebenen Spenderverfahren wird die Trägerfolie daher nicht über ihre gesamte Breite senkrecht zur Bahnführung umgelenkt, sondern zunächst der Länge nach in Streifen geschnitten und dann streifenweise umgelenkt, wobei jedem der Streifen eine eigene Spenderkante zugeordnet ist, die senkrecht oder unter einem Winkel zur Transportrichtung der Trägerfolie angeordnet sein kann. Verschiedenen Trägerfolienstreifen zugeordnete Spenderkanten können an einem einzigen Umlenkelement ausgebildet sein, beispielsweise in Form eines Schwalbenschwanzes, oder an verschiedenen Umlenkelementen, die in Transportrichtung der Trägerfolie zueinander beabstandet sind. Durch das streifenweise Umlenken der Trägerfolie ist die beim Ablösen vom Pflaster jeweils zu überwindende Haftkraft geringer als beim zuvor beschriebenen Spenderverfahren, so dass auch Pflaster mit einer verhältnismäßig geringen Eigensteifigkeit von einer Trägerfolie auf eine Schutzfolie überführt werden können.

Damit die Pflaster bei der Übergabe ohne Beschädigung und möglichst rückstandslos von der Trägerfolie abgelöst werden können, darf die Haftkraft der Trägerfolie an den Pflastern nicht zu hoch sein. Um dies zu erreichen, werden vorzugsweise Trägerfolien verwendet, die zumindest an der Seite, auf die das Wirkstoffdepot aufgebracht wird, mit einer Silicon-, Fluorpolymer- oder Fluorsiliconbeschichtung versehen ist. Ferner muss die Trägerfolie eine ausreichende mechanische Stabilität aufweisen, damit während des Produktionsprozesses keine Faltenbildung auftritt, die zu Inhomogenitäten der auf die Fläche bezogenen Wirkstoffkonzentration führen könnte. Zur Herstellung transdermaler therapeutischer Systeme geeignete Trägerfolien sind entsprechend teuer, so dass das in den beschriebenen Spenderverfahren in Kauf genommene Opfern der Trägerfolie in deutlich erhöhten Herstellungskosten resultiert.

Es ist daher wünschenswert, transdermale therapeutische Systeme mit sich überlappenden Schutzfolienteilen bzw. mit sich in Material oder Oberflächengestaltung unterscheidenden Schutzfolienteilen mit geringerem Materialaufwand herstellen zu können.

Ausführungen entsprechender Herstellungsverfahren für transdermale therapeutische Systeme umfassen Schritte zum Aufbringen eines bahnförmigen Wirkstoffdepots auf eine bahnförmige Trägerfolie so, dass das Wirkstoffdepot haftklebend an der Trägerfolie angebracht ist, zum Durchtrennen des bahnförmigen Wirkstoffdepots entlang linienförmiger Geometrien zur Abgrenzung von zumindest mehreren ersten Wirkstoffdepotabschnitten, zum Einbringen eines Schlitzes in die bahnförmige Trägerfolie von deren nicht mit dem Wirkstoffdepot belegten Seite, wobei der Schlitz zwei in Längsrichtung der Trägerfolie nebeneinander angeordnete Trägerfolienstreifen so definiert, dass die ersten Wirkstoffabschnitte jeweils auf beiden Trägerfolienstreifen aufliegen, zum Bewegen der geschlitzten Trägerfolie entlang deren Längsrichtung und Umlenken von einem ersten der Trägerfolienstreifen an einer ersten Umlenkeinrichtung in Richtung weg von der mit dem Wirkstoffdepot belegten Seite der Trägerfolie, und zum Zuführen eines Schutzfolienstreifens nach dem Umlenken des ersten Trägerfolienstreifens so, dass der Schutzfolienstreifen parallel zum zweiten Trägerfolienstreifen angeordnet die nicht vom zweiten Trägerfolienstreifen bedeckten Bereiche der auf diesem befindlichen ersten Wirkstoffdepotabschnitte bedeckt.

Ausführungen von Produktionsanlagen zur materialnutzenden Herstellung transdermaler therapeutischer Systeme umfassen eine Vorrichtung zum Ersetzen eines Trägerfolienstreifens durch einen Schutzfolienstreifen, wobei die Vorrichtung eine erste Umlenkeinrichtung zum Umlenken eines ersten Trägerfolienstreifens einer relativ zur Umlenkeinrichtung bewegten Trägerfolie aufweist, wobei eine erste Seite der Trägerfolie mit Wirkstoffabschnitten belegt und die Umlenkeinrichtung an der dieser gegenüber angeordneten zweiten Seite der Trägerfolie so angeordnet ist, dass das Umlenken des ersten Trägerfolienstreifens in eine Richtung weg von der ersten Seite der Trägerfolie erfolgt, sowie eine Schutzfolienstreifen-Zufuhreinrichtung, die bezüglich der Bewegungsrichtung der Trägerfolie auf die erste Umlenkeinrichtung folgend angeordnet und so ausgebildet ist, dass der Schutzfolienstreifen parallel zu einem zweiten Trägerfolienstreifen der Trägerfolie angeordnet und zuvor von dem ersten Trägerfolienstreifen bedeckte Bereiche der ersten Wirkstoffabschnitte bedeckt.

Das angegebene Verfahren nutzt zumindest einen Teil der Trägerfolie als Schutzfolie und verringert so die mit Wirkstoffen kontaminierte Abfallmenge. Das Verfahren lässt sich mit der ebenfalls angegebenen Vorrichtung effektiv ausführen.

Vorteilhaft ist die erste Umlenkeinrichtung von einem Keil gebildet, dessen an die Umlenkkante grenzenden Führungsflächen einen Winkel von weniger als 180° und insbesondere von weniger als 90° einschließen, und der so angeordnet ist, dass die Umlenkkante parallel zu den Oberflächen der Trägerfolie und in einem schrägen oder senkrechten Winkel zur Bewegungsrichtung der Trägerfolie ausgerichtet ist. Ein wie beschrieben ausgeführter Keil ermöglicht eine an die Steifigkeit des ersten Trägerfolienstreifens und der ersten Wirkstoffdepotabschnitte angepasste Umlenkung innerhalb eines kurzen Bereichs.

Um die Reibungsbelastung des ersten Trägerfolienstreifens zu minimieren, ist bei Ausführungsformen die erste Umlenkeinrichtung vorteilhaft von einer Umlenkrolle gebildet, deren Drehachse parallel zu den Oberflächen der Trägerfolie und in einem vorzugsweise senkrechten Winkel zur Bewegungsrichtung der Trägerfolie ausgerichtet ist.

Der Schutzfolienstreifen grenzt bei Ausführungsformen des Verfahrens mit einer seiner Seitenkanten an die von dem Schlitz gebildete Seitenkante des zweiten Trägerfolienstreifens an, so dass der Schutzfolienstreifen im Zusammenwirken mit dem zweiten Trägerfolienstreifen die haftklebende Seite der ersten Wirkstoffdepotabschnitte vollständig abdeckt. Bei anderen Ausführungsformen überlappt der Schutzfolienstreifen den zweiten Trägerfolienstreifen, wodurch das Abziehen der Schutzfolie von einem transdermalen therapeutischen System vorteilhaft erleichtert und ein Austreten von kaltfließenden Bestandteilen der ersten Wirkstoffdepotabschnitte wirkungsvoll unterbunden wird.

Für ein Umlenken des ersten Trägerfolienstreifens mit geringem Kraftaufwand ist der Schlitz vorzugsweise so ausgeführt, das er die Trägerfolie durchtrennt. Bei Herstellung von transdermalen therapeutischen Systemen mit kaltfließenden Wirkstoffdepotbestandteilen durchtrennt der Schlitz die Trägerfolie vorteilhaft nur soweit, dass in dieser eine Sollbruchstelle ausgebildet wird, die erst beim Umlenkvorgang aufgetrennt wird.

Bei großflächigen transdermalen therapeutischen Systemen werden vorzugsweise zwei Schlitze so in die Trägerfolie eingebracht, dass die Schlitze drei in Längsrichtung der Trägerfolie nebeneinander angeordnete Trägerfolienstreifen definieren, und die ersten Wirkstoffabschnitte jeweils auf allen drei Trägerfolienstreifen aufliegen. Bei zweckmäßigen Ausgestaltungen hiervon ist der erste Trägerfolienstreifen zwischen dem zweiten und dem dritten Trägerfolienstreifen angeordnet und kann daher bei Bedarf durch einen die äußeren Trägerfolienstreifen überlappenden zentralen Schutzfolienstreifen ausgewechselt werden.

Bei bevorzugten Ausführungsformen erfolgt das Zuführen des Schutzfolienstreifens über eine zweite Umlenkeinrichtung, so dass die zuzuführende Schutzfolie abseits der Trägerfolienbahn gehaltert werden kann. Die Vorrichtung zum Ersetzen eines Trägerfolienstreifens durch einen Schutzfolienstreifen weist hierzu zweckmäßig eine Schutzfolienstreifen-Zufuhreinrichtung auf, die eine zweite Umlenkeinrichtung umfasst. Die zweite Umlenkeinrichtung kann zweckmäßig einen stumpfwinkeligen oder spitzwinkeligen Keil und/oder eine Umlenkrolle umfassen.

Bei Ausführungsformen des Verfahrens werden beim Durchtrennen des bahnförmigen Wirkstoffdepots entlang linienförmiger Geometrien ferner ein zweiter Wirkstoffdepotabschnitt oder mehrere zweite Wirkstoffdepotabschnitte abgegrenzt, und dieser oder diese anschließend von der Trägerfolie vor dem Umlenken des ersten Trägerfolienstreifens abgezogen. Hierdurch wird sichergestellt, dass der umgelenkte erste Trägerfolienstreifen keine Wirkstoffdepotabschnitte mehr mit sich führt.

Bei vorteilhaften Ausführungsformen wird ein Schutzfolienstreifen verwendet, der sich zum leichteren Entfernen der Schutzfolienteile von dem Trägerfolienstreifen in der Art des ihn bildenden Materials und/oder in der Art der Oberflächenbeschichtung an zumindest der zu den ersten Wirkstoffdepotabschnitten weisenden Seite unterscheidet. Bei besonders bevorzugten Ausführungsformen wird der Schutzfolienstreifen von dem zuvor umgelenkten Trägerfolienstreifen gebildet.

Zur vollständigen Nutzung der Trägerfolie umfassen Ausführungsformen der Vorrichtung ferner eine Rückführeinrichtung, die zum Zurückführen des umgelenkten Trägerfolienstreifens zur Schutzfolienstreifen-Zufuhreinrichtung ausgebildet ist. Bei vorteilhaften Ausführungsformen hiervon umfasst die Rückführeinrichtung zwei Umlenkrollen, deren Drehachsen schräg zueinander angeordnet sind und so den ersten Trägerfolienstreifen überlappend zum zweiten Trägerfolienstreifen zurückführen.

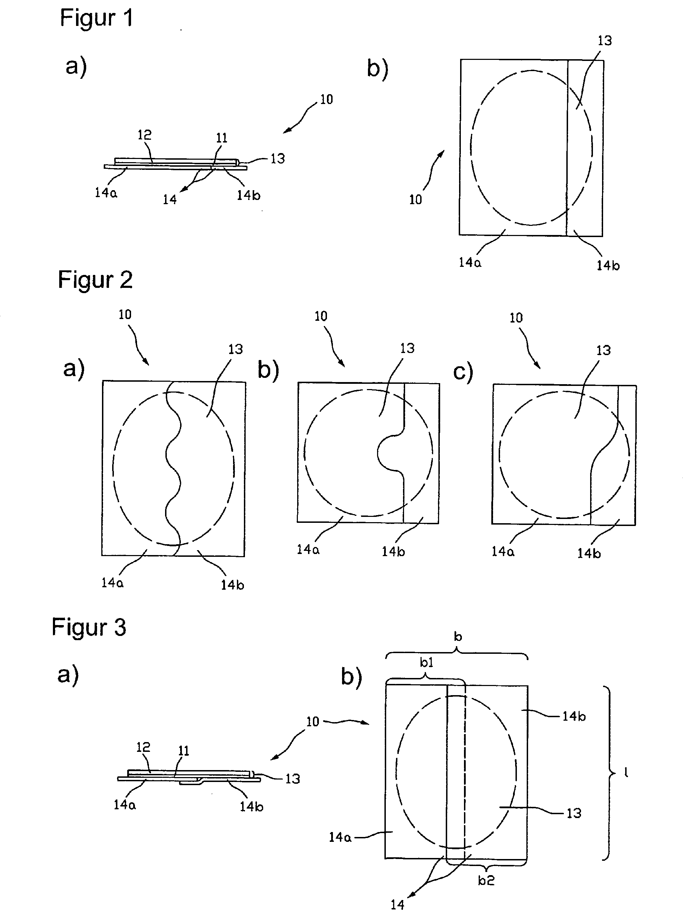

Weitere Merkmale der Erfindung ergeben sich aus der nachfolgenden Beschreibung von Ausführungsbeispielen in Verbindung mit den Ansprüchen sowie den beiliegenden Figuren. Es sei darauf hingewiesen, dass die Erfindung nicht auf die Ausführungsformen der beschriebenen Ausführungsbeispiele beschränkt, sondern durch den Umfang der beiliegenden Patentansprüche bestimmt ist. Insbesondere können bei erfindungsgemäßen Ausführungsformen die bei den nachstehend erläuterten Ausführungsbeispielen angeführten Merkmale in von den Beispielen abweichender Anzahl und Kombination verwirklicht sein. Bei der nachfolgenden Erläuterung einiger Ausführungsbeispiele der Erfindung wird auf die beiliegenden Figuren 1 Bezug genommen, von denen
- Figur 1: ein Beispiel für ein transdermales therapeutisches System mit zweigeteilter Schutzfolie und aneinanderstoßenden Schutzfolienteilen in einer schematischen Querschnitts- sowie Unteransichtsdarstellung veranschaulicht,
- Figur 2: verschiedene beispielhafte Ausgestaltungen von transdermalen therapeutischen Systemen mit zweigeteilter Schutzfolie und aneinanderstoßenden Schutzfolienteilen in schematischen Unteransichtsdarstellungen illustriert,
- Figur 3: ein Beispiel für ein transdermales therapeutisches System mit zweigeteilter Schutzfolie und sich überlappenden Schutzfolienteilen in einer schematischen Querschnitts- und Unteransichtsdarstellung zeigt,
- Figur 4: eine erste Vorrichtung zum Abziehen eines Trägerfolienstreifens und anschließendem Aufbringen eines Schutzfolienstreifens anhand einer schematisierten Perspektivansicht vorstellt,
- Figur 5: eine weitere Vorrichtung zum Abziehen eines Trägerfolienstreifens und anschließendem Aufbringen eines Schutzfolienstreifens anhand einer schematisierten Draufsicht zeigt, und
- Figur 6: in einer schematischen Perspektivansicht die wesentlichen Elemente einer weiteren Vorrichtung veranschaulicht, die zum Abziehen eines Trägerfolienstreifens und anschließendem Aufbringen desselben als überlappenden Schutzfolienstreifen ausgebildet ist.

In den Figuren werden gleiche oder ähnliche Bezugszeichen für funktionell gleichwertige oder ähnliche Charakteristiken unabhängig von speziellen Ausführungsformen verwendet.

Die Illustrationen von Figur 1 veranschaulichen den prinzipiellen Aufbau eines im Folgenden auch Pflaster 10 genannten transdermalen therapeutischen Systems 10 mit zweigeteilter Schutzfolie 14, wobei die beiden Schutzfolienteile 14 a und 14b bei der dargestellten Ausführungsform ohne Überlappung über ihre gesamte Länge unmittelbar aneinander angrenzen. Darstellung a) von Figur 1 gibt das transdermale System 10 im Querschnitt, Darstellung b) als Unteransicht wieder. Das dargestellte Pflaster 10 weist ein Wirkstoffdepot 11 auf, das an der nicht zur Wirkstoffübertragung in die Haut eines Patienten vorgesehenen Rückseite von einer Rückschicht 12 abgedeckt ist. Die Rückschicht 12 ist mit dem Wirkstoffdepot 11 in Form eines Laminats 13 fest verbunden und wird von einer wirkstoffundurchlässigen Folie gebildet. Gegenüber der Rückschicht 12, an der für die Verabreichung von Wirkstoff bzw. Wirkstoffen zum Kontakt mit der Haut vorgesehenen Seite, ist das Wirkstoffdepot 11 von einer aus den beiden Schutzfolienteilen 14a und 14b gebildeten wirkstoffundurchlässigen Schutzfolie 14 bedeckt. Zur Applikation eines wie dargestellten Pflasters 10 werden die beiden Schutzfolienteile 14a und 14b nacheinander entfernt und der dadurch jeweils freigelegte selbstklebende Teil der hautseitigen Oberfläche des Wirkstoffdepots 11 auf die Haut eines Patienten aufgebracht. Bei Patienten kann es sich sowohl um menschliche als auch tierische Wesen handeln.

Die beiden Schutzfolienteile 14a und 14b stoßen bei den dargestellten Ausführungsformen an einem ihrer Ränder unmittelbar aneinander, so dass sie eine zusammenhängende, geschlossene Fläche bilden. Die Lage des von den Schutzfolienteilen 14a und 14b abgedeckten Laminats 13 ist in Darstellung b) von Figur 1 mittels einer gestrichelten Linie veranschaulicht. Bei der gezeigten Ausführungsform sind die lateralen Abmessungen des Laminats 13 kleiner als die der Schutzfolie 14, wodurch die Schutzfolienteile 14a und 14b beidseitig ohne Gefahr einer Berührung eines wirkstoffhaltigen Teils des Laminats 13 angefasst werden können. Ein entsprechender Überstand der Schutzfolie erleichtert zwar die Applikation eines transdermalen therapeutischen Systems 10, ist jedoch nicht immer erforderlich. Das Abziehen von Schutzfolienteile 14a und 14b ohne Überstand kann durch einfaches Biegen des Pflasters 10 im Bereich der Stoßstelle beider Schutzfolienteile bewirkt werden. Ferner besteht bezüglich der Formen von Wirkstoffdepot bzw. Laminat und Schutzfolie ein großer Gestaltungsspielraum, wobei die Formgestaltung an bestimmte Applikationsbereiche angepasst aber beispielsweise auch als Identifikationshinweis für bestimmte mit dem jeweiligen transdermalen therapeutischen System 10 zu verabreichende Wirkstoffe bzw. Wirkstoffkombinationen erfolgen kann. Unter der Voraussetzung, dass die Schutzfolie 14 die hautseitige Oberfläche des Wirkstoffdepots stets vollständig abdeckt, bestehen auch für die Formgestaltung von Schutzfolien keine Einschränkungen.

Beispiele für transdermale therapeutische Systeme 10 mit im Vergleich zur Ausführungsform der Figur 1 unterschiedlichen Ausprägungen von Schutzfolie bzw. Wirkstoffdepot sind in den Darstellungen von Figur 2 illustriert. Im Unterschied zu den Ausführungsformen nach Figur 1 und Figur 2a), weist beispielsweise das Wirkstoffdepot von Ausführungsformen gemäß den Darstellungen von Figur 2b) und c) eine runde Form auf. Die zwischen den nebeneinander angeordneten Schutzfolienteilen 14a und 14b ausgebildete Trennlinie bzw. Stoßstelle ist bei dem in Figur 2a) veranschaulichten Ausführungsbeispiel als Wellenlinie, bei dem in Figur 2b) dargestellten Beispiel als gerade, mittig mit einer Ausbuchtung versehene Linie, und bei dem in Figur 2c) vorgestellten Pflaster 10 als zwei durch einen geschwungenen Übergang verbundene, zueinander versetzte gerade Linien ausgebildet.

Um ein Entfernen der Schutzfolienteile 14a und 14b vom Wirkstoffdepot 11 zu erleichtern, können die beiden Schutzfolienteile 14a und 14b voneinander abweichende physikalische Eigenschaften aufweisen, die sich auf die Haftkraft der Schutzfolienteile auf der selbstklebenden Oberfläche des Wirkstoffdepots bzw. auf die Effektivität auswirken, mit der eine zum Überwinden der Haftkraft erforderliche Trennkraft beim Abziehen der Schutzfolienteile aufgebracht wird.

Bei einem zum Kaltfließen neigenden Wirkstoffdepot wird ein Austreten von Bestandteilen des Depots über die Trennstelle zwischen den Schutzfolienteilen häufig wie in Figur 3 dargestellt durch Ausbilden der Schutzfolie 14 mit zwei sich überlappenden Schutzfolienteilen 14a und 14b unterbunden. Die Summe der Breiten b1 und b2 der beiden Schutzfolienteile ist hierbei größer als die Breite b der resultierenden Schutzfolie 14, wobei die beiden Schutzfolienteile in der Regel dieselbe Länge 1 aufweisen.

Das transdermale therapeutische System 10 kann sowohl als Matrixsystem als auch als Reservoirsystem ausgeführt sein. Das von einer Matrix oder einem Reservoir gebildete Wirkstoffdepot ist auf einer Seite mit einer wirkstoffundurchlässigen Rückschicht 12 bedeckt. Die dieser Seite gegenüberliegende Applikationsseite des Wirkstoffdepots dient dem Herstellen eines Kontakts mit der Haut eines Patienten. Die Applikationsseite ist haftklebend ausgebildet, worunter zu verstehen ist, dass das Wirkstoffdepot entweder selbst haftklebend ausgebildet, hautseitig mit einer selbsthaftenden Schicht versehen, oder von einem Kleberand eingefasst ist.

Bei Ausführung als Matrixsystem besteht das Wirkstoffdepot 11 vorzugsweise aus einer den Wirkstoff enthaltenden Matrix auf Basis eines selbsthaftenden Klebstoffs. Zur Ausbildung der Matrix eignen sich alle gewöhnlich für transdermale therapeutische Systeme geeigneten Materialien, wie z. B. Homo- und Copolymere von (Meth)acrylaten, Polyvinylether, Polyisobutylene, Polyisoprenkautschuk, Styrol-Butadien-Copolymere, Styrol-Butadien-StyrolCopolymere. Von den (Meth)acrylat-Copolymeren können beispielsweise die Copolymere von Alkylacrylaten und/oder Alkylmethacrylaten und weiteren ungesättigten Monomeren genannt werden, wie Acrylsäure, Methacrylsäure, Acrylamid, Dimethylacrylamind, Dimethylaminoethylacrylamid, Acrylnitril und/oder Vinylacetat angegeben werden. Grundsätzlich ist es natürlich auch möglich, ein selbsthaftendes Matrixsystem hautseitig mit einer weiteren haftklebenden Schicht zu versehen, beispielsweise um die Haftung an der Haut noch weiter zu verbessern.

Bei Ausführung des transdermalen therapeutischen Systems 10 als Reservoirsystem ist der Wirkstoff gewöhnlich in einer Flüssigkeit oder einem Gel enthalten, wobei das Wirkstoffreservoir hautseitig eine Membran aufweist, durch die die Abgabe des Wirkstoffes an die Haut kontrolliert wird. Die hautseitige Oberfläche des Laminats 13 ist bei Reservoirsystemen vorzugsweise mit einem selbsthaftenden Klebstoff beschichtet, um die Haftung der Schutzfolie darauf und die Fixierung des Pflasters auf der Haut zu ermöglichen. Alternativ kann eine üblicherweise als Overtape bezeichnete, das Laminat überdeckende, an der Hautseite mit einem selbsthaftenden Kleber versehene Deckschicht verwendet werden, die das Reservoir innerhalb eines Kleberands einfasst.

Als Schutzfolienmaterialien eignen sich beispielsweise Polyethylene, wie HDPE, Polypropylene, Polyester, Polysiloxane, Polyethylenterephthalate, Polyvinylchloride oder Polyurethane, gegebenenfalls in Form von Laminaten. Geeignete Schutzfolien sind beispielsweise von der Firma 3M unter der Markenbezeichnung Scotchpak^{®} (wie Scotchpak^{®} 1020, 9736, 9742, 9744), von der Firma Siliconature z. B. unter der Bezeichnung Silthene oder Silphan oder von der Firma Mitsubishi unter der Markenbezeichnung Hostaphan^{®} (z. B. GN, RD) erhältlich.

Bei Verfahren zur Herstellung von wie oben beschriebenen transdermalen therapeutischen Systemen 10 wird ein bahnförmiges Wirkstoffdepot auf eine bahnförmige Trägerfolie 21 aufgebracht. Das bahnförmige Wirkstoffdepot ist dabei höchstens so breit wie die Trägerfolienbahn 21. Die Herstellung des Wirkstoffdepots kann z. B. durch Aufbringen einer entsprechenden Beschichtungsmasse direkt auf die Trägerfolienbahn 21 unter Verwendung eines Schlitzgießers erfolgen. Die Beschichtungsmasse kann jedoch auch zunächst auf eine folienbahnförmige Rückschicht aufgetragen werden, bevor das Wirkstoffdepot dann mit der nicht von der Rückschicht bedeckten Seite auf die Trägerfolie 21 aufgebracht wird. Wird das Wirkstoffdepot unmittelbar auf die bahnförmige Trägerfolie aufgebracht, so wird die Rückschichtfolie zweckmäßigerweise noch vor einer Abgrenzung von für einzelne transdermale therapeutische Systeme 10 bestimmten Bereichen auf die freie Oberfläche der Wirkstoffdepotbahn aufgebracht.

Die Abgrenzung der für die einzelnen Pflaster 10 bestimmten Bereiche der Wirkstoffdepotbahn erfolgt üblicherweise durch Stanzen einer linienförmigen Kontur in das bahnförmige Wirkstoffdepot so, dass die Wirkstoffdepotbahn entlang einer die Pflasterkonturen festlegenden Geometrie durchtrennt wird. Vorzugsweise aber nicht notwendigerweise sind die hierdurch festgelegten Pflasterbereiche räumlich voneinander isoliert und der Bereich der Wirkstoffdepotbahn außerhalb der Pflasterbereiche ist zusammenhängend ausgebildet. Er bildet dann ein sogenanntes Gitter oder Stanzgitter, das zur Vereinzelung der Pflasterbereiche in einem Abgittern genannten kontinuierlichen Verfahrensschritt von der Trägerfolie 21 abgezogen werden kann.

Zum Erzielen einer zweigeteilten Schutzfolie 14 unter Verwendung der Trägerfolienbahn 21 wird in diese ein Schlitz 29 eingebracht, der die Trägerfolie 21 entweder vollständig oder zumindest soweit durchtrennt, dass eine ohne nennenswerten Kraftaufwand auftrennbare Sollbruchstelle entsteht. Das Schlitzen kann mithilfe von stehenden oder rotierenden Messern erfolgen, die in die an ihnen vorbeigeführte Trägerfolienbahn 21 einen in Längsrichtung der Trägerfolie 21 orientierten, zumeist geraden Schlitz einbringen. Bei anderen Verfahren wird der Schlitz durch Stanzen mit einer Schneideinrichtung eingebracht, wobei die Trägerfolie hierzu vorzugsweise diskontinuierlich bewegt wird. Letzteres Verfahren eignet sich insbesondere für Schlitzformen mit schräg oder quer zur Bewegungsrichtung der Trägerfolie 21 orientierten Bereichen, wie z. B. schlangenlinienförmigen Schlitzen. Das Schlitzen der Trägerfolienbahn 21 wird gewöhnlich vor einem Ausbilden von Pflasterkonturen in der Wirkstoffdepotbahn vorgenommen.

Der bisher beschriebene Verfahrensablauf eignet sich für die Herstellung von transdermalen therapeutischen Systemen, deren Schutzfolienteile sich nicht überlappen und abgesehen von Größe und Randgestaltung identisch sind. Zur Herstellung transdermaler therapeutischer Systeme 10 mit sich überlappenden Schutzfolienteilen bzw. Schutzfolienteilen aus unterschiedlichen Materialien oder mit unterschiedlicher Oberflächengestaltung werden die vereinzelten Pflasterbereiche von der - nicht notwendigerweise - geschlitzten Trägerfolie 21 abgenommen und auf eine den jeweiligen Spezifikationen genügende wenigstens zweiteilige Schutzfolienbahn übertragen. Das Übertragen wird gegenwärtig mithilfe einer Vakuumwalze oder einem der zuvor beschriebenen Spenderverfahren vorgenommen werden, wobei die Trägerfolie 21 selbst stets vollständig geopfert wird.

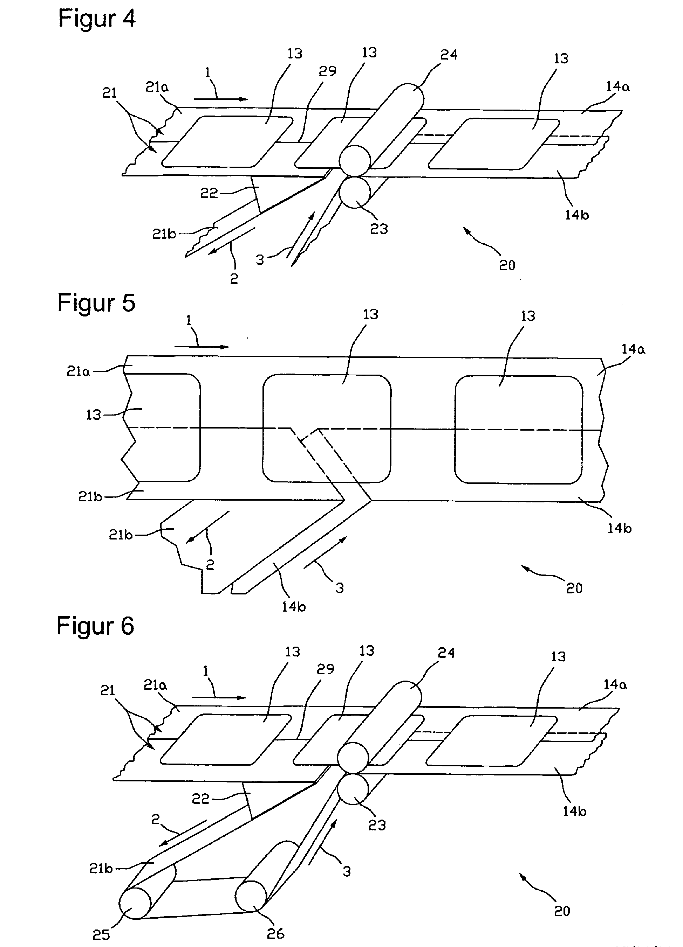

Figur 4 illustriert einen Teil 20 einer Produktionsanlage zur weniger materialintensiven Herstellung von transdermalen therapeutischen Systemen 10 mit z. B. sich überlappenden Schutzfolienteilen 14a und 14b bzw. mit Schutzfolienteilen, die sich in Material bzw. Oberflächengestaltung unterscheiden. Bei dem dargestellten Produktionsablauf wird zumindest ein Teil der Trägerfolienbahn 21 zur Ausbildung der Schutzfolie 14 weiterverwendet. Die Prinzipdarstellung von Figur 4 beschränkt sich wie auch die der Figuren 5 und 6 auf die zum Verständnis der vorliegenden Erfindung wesentlichen Komponenten. Auf die Darstellung weiterer für die Herstellung transdermaler therapeutischer Systeme 10 erforderlicher oder geeigneter Komponenten wurde im Hinblick auf eine übersichtliche Darstellung verzichtet. Die zuvor beschriebene Vereinzelung der Pflasterlaminate 13 und das Schlitzen der Trägerfolie 21 erfolgt in Bereichen der Produktionsanlage, die dem dargestellten Teil 20 vorausgehen.

Die bereits in Längsrichtung geschlitzte Trägerfolienbahn 21 wird in der mittels Pfeil 1 angedeuteten Richtung bewegt und weist voneinander beabstandete Laminate 13 auf, deren Applikationsseite jeweils über den Schlitz hinweg auf beiden Trägerfolienstreifen 21a und 21b aufliegt. Unterhalb, d. h. an der nicht die Pflasterlaminate 13 tragenden Seite der Trägerfolie 21 ist eine erste Umlenkeinrichtung 22 angeordnet, an der der erste Trägerfolienstreifen 21b in die durch den Pfeil 2 angedeutete Richtung umgelenkt wird. Der zweite Trägerfolienstreifen 21 a wird nicht umgelenkt, sondern bewegt sich auch dann weiter in die durch Pfeil 1 gekennzeichnete Richtung, wenn sich die erste Umlenkeinrichtung 22 bis unter diesen ersten Streifen erstreckt. Beim Umlenken des ersten Trägerfolienstreifens 21b löst sich dieser von dem darauf befindlichen Laminat 13 ab. Da das Laminat 13 mit dem übrigen Teil weiterhin auf dem zweiten Trägerfolienstreifen 21a haftet, wird es auf diesem in Richtung des Pfeils 1 weitertransportiert.

Bei der in Figur 4 veranschaulichten Ausführungsform ist die erste Umlenkeinrichtung 22 keilförmig ausgebildet, so dass der umgelenkte Bereich des ersten Trägerfolienstreifens 21b mit dem noch nicht umgelenkten Bereich einen spitzen Winkel einschließt. Die Umlenkkante der Umlenkeinrichtung 22 ist hierbei bevorzugt gerundet ausgeführt, um keine Schnittkräfte auf den Folienstreifen 21b auszuüben, die zu dessen Beschädigung oder zu dessen Einreißen führen könnten. Beim spitzwinkeligen Umlenken des ersten Trägerfolienstreifens 21b um eine gerundete Kante wird dieser von der Unterseite der Pflasterlaminate 13 im Wesentlichen senkrecht und damit mit maximal möglicher Trennkraft abgezogen. Da der zweite Trägerfolienstreifen 21a nicht mit umgelenkt wird, bleiben die Pflasterlaminate 13 weiter darauf haften, wodurch das Abziehen des ersten Trägerfolienstreifens 21 b zusätzlich unterstützt wird. Außerdem stützt der zweite Trägerfolienstreifen 21a die Pflasterlaminate 13 während des Abziehens des ersten Trägerfolienstreifens 21 b, so dass auch Pflasterlaminate 13 mit geringer Eigensteifigkeit verwendet werden können.

Bei Pflasterlaminaten mit ausreichender Eigensteifigkeit bzw. bei Trägerfolien 21 mit geringer Haftung auf den Pflasterlaminaten kann zum Abziehen des ersten Trägerfolienstreifens 21b auch ein stumpfer Umlenkwinkel von weniger als 180° verwendet werden. Entsprechende Umlenkwinkel empfehlen sich insbesondere bei steifen Trägerfolien, deren Umlenkung einen hohen Kraftaufwand erfordert, wodurch die Gleitreibung des Streifens 21b auf der Umlenkeinrichtung 22 erhöht und deren Verschleiß beschleunigt wird.

Zur Verminderung eines durch Gleitreibung bewirkten Verschleißes der ersten Umlenkeinrichtung 22 kann diese statt wie gezeigt als Kante in Form einer der Rolle 23 von Figur 4 ähnlichen Umlenkrolle ausgebildet sein.

Bei der in Figur 4 dargestellten Umlenkeinrichtung 22 verläuft die Umlenkkante in einem rechten Winkel zu der durch Pfeil 1 gekennzeichneten Transportrichtung der Trägerfolie 21. Bei einer solchen Anordnung wird der erste Trägerfolienstreifen über die gesamte Breite von dem über die Kante geführten Pflasterlaminat 13 abgezogen, und die dabei ausgeübte Zugkraft kann ein hochflexibles oder elastisches Pflaster unzweckmäßig verformen. Um die Zugkraft zu vermindern und das Pflasterlaminat 13 während des Abziehens weiterhin an seinem äußeren Rand zu stützen, wird die Umlenkkante bei vorteilhaften Ausführungsformen unter einem Winkel schräg zur Laufrichtung der Trägerfolie 21 so angeordnet, dass der erste Trägerfolienstreifen 21 b zuerst am Schlitz und nachfolgend erst an den äußeren Bereichen abgezogen wird. Eine entsprechende Anordnung der Umlenkkante ist in Figur 5 in Draufsicht anschaulich vorgestellt, wobei auf die Wiedergabe weiterer Elemente der Vorrichtung 20 zugunsten einer klaren Darstellung verzichtet wurde.

Nach dem Umlenken des ersten Trägerfolienstreifens 21b wird der hierbei freigelegte Bereich des Pflasterlaminats 13 mit einem Schutzfolienstreifen 14b bedeckt. Der Schutzfolienstreifen 14b wird über eine zweite Umlenkeinrichtung 23 zugeführt, die in der in Figur 4 dargestellten Ausführungsform von einer Umlenkrolle 23 gebildet wird. Die Drehachse der dargestellten Umlenkrolle 23 ist im rechten Winkel zur Laufrichtung des zweiten Trägerfolienstreifens 21 a angeordnet. Die Drehachse ist parallel zur Unterseite des zweiten Trägerfolienstreifens 21 a bzw. Schutzfolienstreifens 14a ausgerichtet und grenzt an diesen an bzw. weist, falls dieser von dem zugeführten Schutzfolienstreifen 14b überlappt wird, einen an die Dicke des Schutzfolienstreifens 14b angepassten Abstand zur Unterseite des zweiten Trägerfolienstreifens 21a auf. Zum Sicherstellen einer ganzflächigen Haftung der Pflasterlaminate 13 auf den Schutzfolienstreifen 14a und 14b kann, wie in Figur 4 zu sehen ist, eine Andruckrolle 24 verwendet werden.

Der zugeführte Schutzfolienstreifen 14b kann aus einem von der Trägerfolie 21 verschiedenen Material gebildet sein. Beispielsweise kann ein flexibleres, d. h. leichter biegbares, Material verwendet werden, oder auch ein Material, dass eine andere Dicke bzw. Folienstärke aufweist. Ferner kann der Schutzfolienstreifen 14b an der zu den Pflasterlaminaten 13 weisenden Seite eine Oberflächenbeschichtung aufweisen, die eine zur Trägerfolie unterschiedliche Haftung auf den selbstklebenden Oberflächen der Laminate 13 bewirkt. Schließlich kann der Schutzfolienstreifen 14b bzw. eine oder beide seiner Oberflächen eine dreidimensionale Strukturierung aufweisen, über die die Griffigkeit des Streifens 14b bzw. dessen Kontaktfläche zum Haftkleber modifiziert werden. Zur besseren Handhabung von wie beschrieben hergestellten transdermalen therapeutischen Systemen wird der Schutzfolienstreifen 14b so über die zweite Umlenkeinrichtung 23 zugeführt, dass er den vom zweiten Trägerfolienstreifen 21 a gebildeten anderen Schutzfolienstreifen 14a überlappt. Eine entsprechende Überlappung ist jedoch nicht notwendig.

Die Prinzipdarstellung von Figur 6 illustriert ein Verfahren, bei dem der an der ersten Umlenkeinrichtung 22 von den Pflasterlaminaten 13 abgezogene erste Trägerfolienstreifen 21b über zwei schräg zueinander ausgerichtete Umlenkrollen 25 und 26 bezüglich der durch Pfeil 1 illustrierten Transportrichtung der Trägerfolie 21 seitlich versetzt und anschließend den Pflasterlaminaten 13 über die zweite Umlenkeinrichtung 23 als Schutzfolienstreifen 14b wieder zugeführt wird. Durch das seitliche Versetzen des Streifens 21b wird ein Überlappen des ersten Schutzfolienstreifens 14a durch den zweiten Schutzfolienstreifen 14b erzielt. Der vorhergehende ganzflächige Kontakt des Trägerfolienstreifens 21 b mit der selbstklebenden Oberfläche des bahnförmigen Wirkstoffdepots hat die Haftfähigkeit des Streifens 21b verändert, wodurch die Haftkraft des abgezogenen und anschließend wieder zugeführten Schutzfolienstreifens 14b auf den selbstklebenden Oberflächen der Pflasterlaminate 13 in der Regel geringer ist, als die des vom zweiten Trägerfolienstreifen 21a gebildeten Schutzfolienstreifens 14a. Ein daraus resultierendes erleichtertes Abziehen dieses Schutzfolienstreifens 14b vereinfacht die Applikation transdermaler therapeutischer Systeme 10 und ermöglicht auch Menschen mit motorischen Störungen eine sichere Anwendung dieser Systeme.

Zur Durchführung der beschriebenen Verfahren weist eine Produktionsanlage zur Herstellung transdermaler therapeutischer Systeme eine Wechselvorrichtung 20 auf, die zum Abziehen eines Streifens 21b einer Pflasterlaminate 13 tragenden Trägerfolie 21 von den Laminaten 13 und zum Anbringen einer Schutzfolie 14b auf die durch das Abziehen freigelegten Bereiche der Pflasterlaminate 13 ausgebildet ist. Ausführungen solcher Wechselvorrichtungen 20 umfassen eine erste Umlenkeinrichtung 22 zum Umlenken des Trägerfolienstreifens 21b, wobei die Umlenkeinrichtung 22 so ausgebildet ist, dass ein darüber geführter Trägerfolienstreifen 14b aus seiner ursprünglichen Bewegungsrichtung in eine Richtung weg von der die Pflasterlaminate 13 tragenden Seite der Trägerfolie 21 abgelenkt wird.

Bei Ausführungsformen weist die Umlenkeinrichtung 22 die Form eines Keils auf, wobei die an die Umlenkkante des Keils grenzenden Flächen einen Winkel von weniger als 180°, und bei bevorzugten Ausführungsformen von weniger als 90° einschließen. Zur Verminderung von lokalen Spitzenbelastungen des Trägerfolienstreifens 21 b an der Umlenkkante ist diese vorzugsweise gerundet ausgeführt, wobei der Rundungsradius an Steifigkeit und Oberflächenbeschaffenheit der Trägerfolie 21 angepasst gewählt und vom Fachmann gegebenenfalls durch Versuche ermittelt wird. Die Umlenkkante kann in einem zur Bewegungsrichtung des Trägerfolienstreifens 21b senkrechten oder schrägen Winkel angeordnet sein. Bei Anordnung im schrägen Winkel schließt die Umlenkkante mit der an den anderen Trägerfolienstreifen 21a angrenzenden Seitenkante des Trägerfolienstreifens 21b einen stumpfen Winkel ein.

Andere Ausführungsformen weisen eine Umlenkeinrichtung 22 in Form einer Umlenkrolle auf, deren Achslage entweder senkrecht oder schräg zur Bewegungsrichtung der Trägerfolie 21 angeordnet sein kann.

Für das Heranführen des Schutzfolienstreifens 14b an die selbstklebende Seite der Pflasterlaminate 13 weist die Wechselvorrichtung 20 vorzugsweise eine weitere Umlenkeinrichtung 23 auf, die ebenfalls als stumpf- oder spitzwinkeliger Keil ausgeführt sein kann, bevorzugt aber in Form einer Umlenkrolle ausgebildet ist. Bei weiterhin bevorzugten Ausführungsformen ist eine auf die Umlenkeinrichtung 23 einwirkende Andruckrolle 24 vorgesehen, die zwischen Umlenkeinrichtung 23 und Andruckrolle 24 hindurchgeführte Pflasterlaminate 13 und Schutzfolienstreifen 14b aufeinander drückt.

Weitere Ausführungsformen von Wechselvorrichtungen 20 weisen eine Einrichtung zum Zurückführen des von der ersten Umlenkeinrichtung 22 umgelenkten Trägerfolienstreifens 21b als Schutzfolienstreifen 14b an die zweite Umlenkeinrichtung 23 auf. Eine solche Rückführeinrichtung umfasst bei bevorzugten Ausführungsformen eine Einrichtung zur Überlappung des rückgeführten Trägerfolienstreifens 14b mit dem anderen Trägerfolienstreifen 14a.

Ein wie beschriebenes Verfahren und eine wie beschriebene Vorrichtung ermöglichen eine materialsparende Herstellung von transdermalen therapeutischen Systemen mit sich überlappenden Schutzfolienteilen und bzw. oder unterschiedlicher Ausprägung der Schutzfolienteile.

## Patentansprüche

1. Verfahren zum Herstellen eines transdermalen therapeutischen Systems (10) mit Schritten zum:
- Aufbringen eines bahnförmigen Wirkstoffdepots auf eine bahnförmige Trägerfolie (21) so, dass das Wirkstoffdepot haftklebend an der Trägerfolie (21) angebracht ist,
- Durchtrennen des bahnförmigen Wirkstoffdepots entlang linienförmiger Geometrien zur Abgrenzung von zumindest mehreren ersten Wirkstoffdepotabschnitten (11),
- Einbringen eines Schlitzes (29) in die bahnförmige Trägerfolie (21) von deren nicht mit dem Wirkstoffdepot belegten Seite, wobei der Schlitz (29) zwei in Längsrichtung (1) der Trägerfolie nebeneinander angeordnete Trägerfolienstreifen (21a, 21b) so definiert, dass die ersten Wirkstoffabschnitte (11) jeweils auf beiden Trägerfolienstreifen (21a, 21 b) aufliegen,
- Bewegen der geschlitzten Trägerfolie (21) entlang deren Längsrichtung (1) und Umlenken von einem ersten (21b) der Trägerfolienstreifen an einer ersten Umlenkeinrichtung (22) in Richtung weg von der mit dem Wirkstoffdepot (11) belegten Seite der Trägerfolie (21), und
- Zuführen eines Schutzfolienstreifens (14b) nach dem Umlenken des ersten Trägerfolienstreifens (21b) so, dass der Schutzfolienstreifen parallel zum zweiten Trägerfolienstreifen (21 a, 14a) angeordnet die nicht vom zweiten Trägerfolienstreifen (21a, 14a) bedeckten Bereiche der auf diesem befindlichen ersten Wirkstoffdepotabschnitte (11) bedeckt.

2. Verfahren nach Anspruch 1, worin der Schutzfolienstreifen (14b) mit einer Seitenkante an die von dem Schlitz gebildete Seitenkante des zweiten Trägerfolienstreifen (21a, 14a) angrenzt.

3. Verfahren nach Anspruch 1, worin der Schutzfolienstreifen (14b) den zweiten Trägerfolienstreifen (14a, 21 a) überlappt.

4. Verfahren nach einem der vorhergehenden Ansprüche, worin in die bahnförmige Trägerfolie (21) von deren nicht mit dem Wirkstoffdepot (11) belegten Seite ein zweiter Schlitz so eingebracht wird, dass die Schlitze drei in Längsrichtung der Trägerfolie nebeneinander angeordnete Trägerfolienstreifen so definieren, dass die ersten Wirkstoffabschnitte (11) jeweils auf allen drei Trägerfolienstreifen aufliegen.

5. Verfahren nach Anspruch 4, worin der erste Trägerfolienstreifen (21b) zwischen dem zweiten und dem dritten Trägerfolienstreifen angeordnet ist.

6. Verfahren nach einem der vorhergehenden Ansprüche, worin das Zuführen des Schutzfolienstreifens (14b) über eine zweite Umlenkeinrichtung erfolgt.

7. Verfahren nach einem der vorhergehenden Ansprüche, worin beim Durchtrennen des bahnförmigen Wirkstoffdepots entlang linienförmiger Geometrien ferner ein zweiter Wirkstoffdepotabschnitt oder mehrere zweite Wirkstoffdepotabschnitte abgegrenzt werden, und dieser oder diese von der Trägerfolie (21) vor dem Umlenken des ersten Trägerfolienstreifens (21b) abgezogen werden.

8. Verfahren nach einem der vorhergehenden Ansprüche, worin der Schutzfolienstreifen (14b) sich von dem zweiten Trägerfolienstreifen (21a, 14a) in der Art des ihn bildenden Materials und/oder in der Art der Oberflächeneigenschaften an der zu den ersten Wirkstoffdepotabschnitten (11) weisenden Seite unterscheidet.

9. Verfahren nach einem der Ansprüche 1 bis 7, worin der Schutzfolienstreifen (14b) von dem zuvor umgelenkten ersten Trägerfolienstreifen (21 b) gebildet wird.

10. Vorrichtung zum Ersetzen eines Trägerfolienstreifens (21b) durch einen Schutzfolienstreifen (14b) für die Herstellung transdermaler therapeutischer Systeme (10), wobei die Vorrichtung (20) Folgendes aufweist:
- eine erste Umlenkeinrichtung (22) zum Umlenken eines ersten Trägerfolienstreifens (21 b) einer relativ zur ersten Umlenkeinrichtung (22) bewegten Trägerfolie (21), wobei eine erste Seite der Trägerfolie (21) mit Wirkstoffabschnitten (11) belegt und die erste Umlenkeinrichtung (22) an der dieser gegenüber angeordneten zweiten Seite der Trägerfolie (21) so angeordnet ist, dass das Umlenken des ersten Trägerfolienstreifens (21b) in eine Richtung weg von der ersten Seite der Trägerfolie (21) erfolgt,
- eine Schutzfolienstreifen-Zufuhreinrichtung (23), die bezüglich der Bewegungsrichtung (1) der Trägerfolie (21) auf die erste Umlenkeinrichtung (22) folgend angeordnet und so ausgebildet ist, dass der Schutzfolienstreifen (14b) parallel zu einem zweiten Trägerfolienstreifen (21 a) der Trägerfolie (21) angeordnet und zuvor von dem ersten Trägerfolienstreifen (21b) bedeckte Bereiche der ersten Wirkstoffabschnitte bedeckt.

11. Vorrichtung nach Anspruch 10, worin die erste Umlenkeinrichtung (22) von einem Keil gebildet ist, dessen an die Umlenkkante grenzenden Führungsflächen einen Winkel von weniger als 180° und insbesondere von weniger als 90° einschließen, und der so angeordnet ist, dass die Umlenkkante parallel zu den Oberflächen der Trägerfolie (21) und in einem schrägen oder senkrechten Winkel zur Bewegungsrichtung (1) der Trägerfolie (21) ausgerichtet ist.

12. Vorrichtung nach einem der Ansprüche 10 und 11, worin die Schutzfolienstreifen-Zufuhreinrichtung (23) einer zweite Umlenkeinrichtung aufweist.

13. Vorrichtung nach Anspruch 12, worin die zweite Umlenkeinrichtung: (23) einen stumpfwinkeligen oder spitzwinkeligen Keil und/oder eine Umlenkrolle umfasst.

14. Vorrichtung nach einem der Ansprüche 10 bis 13, die ferner eine Rückführeinrichtung (25, 26) umfasst, die zum Zurückführen des umgelenkten Trägerfolienstreifens (21) zur Schutzfolienstreifen-Zufuhreinrichtung (23) ausgebildet ist.

15. Transdermales therapeutisches System (10) erhältlich nach dem Verfahren gemäß einem der Ansprüche 1 bis 9.

## Claims

1. Method for producing a transdermal therapeutic system (10), comprising the steps of:
- applying a web-shaped active-ingredient depot to a web-shaped backing film (21) such that the active-ingredient depot is attached to the backing film (21) in a self-adhesive manner,
- cutting the web-shaped active ingredient depot along linear geometriesto delimit at least a plurality of first active-ingredient-depot portions (11),
- making a slit (29) in the web-shaped backing film (21) from the side thereof not covered with the active-ingredient depot, the slit (29) defining two backing-film strips (21a, 21b) which are arranged side by side in the longitudinal direction (1) of the backing film, such that the first active-ingredient portions (11) each lie on the two backing-film strips (21a, 21b),
- moving the slit backing film (21) in the longitudinal direction (1) thereof and deflecting a first (21b) of the backing-film strips on a first deflecting device (22) away from the side of the backing film (21) covered with the active-ingredient depot (11), and
- feeding a protective-film strip (14b) after deflecting the first backing-film strip (21b) such that the protective-film strip which is arranged parallel to the second backing-film strip (21a, 14a) covers the regions of the first active-ingredient-depot portions (11) located on the second backing-film strip (21a, 14a) which are not covered by said second backing-film strip.

2. Method according to claim 1, wherein a lateral edge of the protective-film strip (14b) adjoins the lateral edge, formed by the slit, of the second backing-film strip (21a, 14a).

3. Method according to claim 1, wherein the protective-film strip (14b) overlaps the second backing-film strip (14a, 21 a) .

4. Method according to any of the preceding claims, wherein a second slit is made in the web-shaped backing film (21) from the side thereof not covered with the active-ingredient depot (11) such that the slits define three backing-film strips which are arranged side by side in the longitudinal direction of the backing film such that the first active-ingredient portions (11) each lie on all three backing-film strips.

5. Method according to claim 4, wherein the first backing-film strip (21 b) is arranged between the second and the third backing-film strip.

6. Method according to any of the preceding claims, wherein the protective-film strip (14b) is fed via a second deflecting device.

7. Method according to any of the preceding claims, wherein a second active-ingredient-depot portion or a plurality of second active-ingredient-depot portions are further delimited when cutting the web-shaped active-ingredient depot along linear geometries, and said second portion or portions are removed from the backing film (2 1) before the first backing-film strip (21 b) is deflected.

8. Method according to any of the preceding claims, wherein the protective-film strip (14b) differs from the second backing-film strip (21a, 14a) by the type of material thereof and/or by the type of surface properties on the side facing the first active-ingredient-depot portions (11).

9. Method according to any of claims 1 to 7, wherein the protective-film strip (14b) is formed by the previously deflected first backing-film strip (21b).

10. Apparatus for replacing a backing-film strip (21b) with a protective-film strip (14b) for producing transdermal therapeutic systems (10), wherein the apparatus (20) comprises:
- a first deflecting device (22) for deflecting a first backing-film strip (21 b) of a backing film (21) which is moved relative to the first deflecting device (22), a first side of the backing film (21) being covered with active-ingredient portions (11) and the first deflecting device (22) being arranged on the second side, facing said deflecting device, of the backing film (21) such that the first backing-film strip (21b) is deflected away from the first side of the backing film (21),
- a protective-film-strip feeding device (23), which is arranged downstream of the first deflecting device (22) relative to the movement direction (1) of the backing film (21) and is formed such that the protective-film strip (14b) is arranged parallel to a second backing-film strip (21a) of the backing film (21) and covers regions of the first active-ingredient portions previously covered by the first backing-film strip (21b).

11. Apparatus according to claim 10, wherein the first deflecting device (22) is formed by a wedge, of which the guide surfaces which adjoin the deflecting edge form an angle of less than 180° and more particularly of less than 90°, and which is arranged such that the deflecting edge is oriented parallel to the surfaces of the backing film (21) and at an angle that is oblique or perpendicular to the movement direction (1) of the backing film (2 1).

12. Apparatus according to either claim 10 or claim 11, wherein the protective-film-strip feeding device (23) comprises a second deflecting device.

13. Apparatus according to claim 12, wherein the second deflecting device (23) comprises an obtuse-angled or acute-angled wedge and/or a deflecting roller.

14. Apparatus according to any of claims 10 to 13, which further comprises a return device (25, 26) which is configured to return the deflected backing-film strip (21) to the protective-film-strip feeding device (23).

15. Transdermal therapeutic system (10) which can be obtained by the method according to any of claims 1 to 9.

## Revendications

1. Procédé de fabrication d'un système thérapeutique transdermique (10), comprenant les étapes destinées à :
- appliquer un dépôt de substance active en forme de bande sur une feuille support (21) en forme de bande de telle sorte que le dépôt de substance active est mis en place sur la feuille support (21) par adhérence,
- découper le dépôt de substance active en forme de bande le long de géométries linéaires pour la délimitation d'au moins plusieurs premiers tronçons de dépôt de substance active (11),
- insérer une fente (29) dans la feuille support (21) en forme de bande par son côté non pourvu du dépôt de substance active, la fente (29) définissant deux rubans de feuille de support (2 la, 21b) disposés de façon juxtaposée dans la direction longitudinale (1) de la feuille support de telle sorte que les premiers tronçons de substance active (11) sont respectivement situés sur les deux rubans de feuille de support (21a, 21b),
- déplacer la feuille support (21) fendue le long de sa direction longitudinale (1) et renvoyer un premier ruban (21 b) des rubans de feuille support sur un premier dispositif de renvoi (22) dans une direction s'éloignant du côté de la feuille support (21) pourvu du dépôt de substance active (11), et
- amener un ruban de feuille de protection (14b) après le renvoi du premier ruban de feuille support (21b) de telle sorte que le ruban de feuille de protection disposé parallèlement au deuxième ruban de feuille support (2 la, 14a) couvre les zones des premiers tronçons de dépôt de substance active (11) situés sur le deuxième ruban de feuille support (21 a, 14a) qui ne sont pas couvertes par le deuxième ruban de feuille support.

2. Procédé selon la revendication 1, dans lequel le ruban de feuille de protection (14b) jouxte par une arête latérale l'arête latérale du deuxième ruban de feuille support (21a, 14a) formée par la fente.

3. Procédé selon la revendication 1, dans lequel le ruban de feuille de protection (14b) chevauche le deuxième ruban de feuille support (14a, 21a).

4. Procédé selon une des revendications précédentes, dans lequel une deuxième fente est insérée dans la feuille support (21) en forme de bande par son côté qui n'est pas pourvu du dépôt de substance active (11) de telle sorte que les fentes définissent trois rubans de feuille support disposés de façon juxtaposée dans la direction longitudinale de la feuille support de telle sorte que les premiers tronçons de substance active (11) sont respectivement situés sur les trois rubans de feuille support.

5. Procédé selon la revendication 4, dans lequel le premier ruban de feuille support (21b) est disposé entre le deuxième et le troisième ruban de feuille support.

6. Procédé selon une des revendications précédentes, dans lequel l'amenée du ruban de feuille de protection (14b) est effectuée par le biais d'un deuxième dispositif de renvoi.

7. Procédé selon une des revendications précédentes, dans lequel, lors de la découpe du dépôt de substance active en forme de bande le long de géométries linéaires, un deuxième tronçon de dépôt de substance active ou plusieurs deuxièmes tronçons de dépôt de substance active sont en outre délimités, et ce tronçon ou ces tronçons est ou sont retirés(s) de la feuille support (21) avant le renvoi du premier ruban de feuille support (21b).

8. Procédé selon une des revendications précédentes, dans lequel le ruban de feuille de protection (14b) se distingue du deuxième ruban de feuille support (21a, 14a) par le type du matériau qui le compose et/ou par le type de caractéristiques superficielles sur le côté tourné vers les premiers dépôts de substance active (11).

9. Procédé selon une des revendications 1 à 7, dans lequel le ruban de feuille de protection (14b) est formé du premier ruban de feuille support (21 b) renvoyé antérieurement.

10. Appareil destiné à remplacer un ruban de feuille support (21b) par un ruban de feuille de protection (14b) pour la fabrication de systèmes thérapeutiques transdermiques (10), le dispositif (20) présentant les éléments suivants :
- un premier dispositif de renvoi (22) pour le renvoi d'un premier ruban de feuille support (21 b) d'une feuille support (21) déplacée par rapport au premier dispositif de renvoi (22), un premier côté de la feuille support (21) étant pourvu de tronçons de substance active (11), et le premier dispositif de renvoi (22) étant disposé sur le deuxième côté de la feuille support (21) qui est disposé en face du premier dispositif de renvoi de telle sorte que le renvoi du premier ruban de feuille support (21b) s'effectue dans une direction qui s'éloigne du premier côté de la feuille support (21),
- un dispositif d'amenée de ruban de feuille de protection (23) qui, par rapport à la direction de mouvement (1) de la feuille support (21), est disposé à la suite du premier dispositif de renvoi (22) et est constitué de telle sorte que le ruban de feuille de protection (14b) est disposé parallèlement à un deuxième ruban de feuille support (21a) de la feuille support (21) et couvre des zones des premiers tronçons de substance active couvertes antérieurement par le premier ruban de feuille support (21b).

11. Appareil selon la revendication 10, dans lequel le premier dispositif de renvoi (22) est formé d'un coin dont les surfaces de guidage limitrophes de l'arête de renvoi forment un angle inférieur à 180° et en particulier inférieur à 90°, et qui est disposé de telle sorte que l'arête de renvoi est orientée parallèlement aux surfaces de la feuille support (21) et en formant un angle oblique ou perpendiculaire par rapport à la direction de mouvement (1) de la feuille support (21).

12. Appareil selon une des revendications 10 et 11, dans lequel le dispositif d'amenée de ruban de feuille de protection (23) présente un deuxième dispositif de renvoi.

13. Appareil selon la revendication 12, dans lequel le deuxième dispositif de renvoi (23) comprend un coin à angle obtus ou à angle aigu et/ou un galet de renvoi.

14. Appareil selon une des revendications 10 à 13, qui comprend en outre un dispositif de retour (25, 26) qui est constitué pour ramener vers le dispositif d'amenée de ruban de feuille de protection (23) le ruban de feuille support (21) renvoyé.

15. Système thérapeutique transdermique (10) pouvant être obtenu suivant le procédé selon une des revendications 1 à 9.
